(19) Europäisches Patentamt
European Patent Office
Office européen des brevets

(11) Publication number: 0 195 097 B1

(12) # EUROPEAN PATENT SPECIFICATION

(45) Date of publication of the patent specification: **28.10.87**

(51) Int. Cl.4: **C 07 C 59/68**, C 07 C 69/736, C 07 C 57/42, C 07 C 69/618, C 07 C 51/347, C 07 C 67/30, A 61 K 31/19, A 61 K 31/215

(21) Application number: **85103186.4**

(22) Date of filing: **19.03.85**

(54) **Arachidonic acid analogues, processes for their preparation and their use in medicine.**

(43) Date of publication of application:
**24.09.86 Bulletin 86/39**

(45) Publication of the grant of the patent:
**28.10.87 Bulletin 87/44**

(84) Designated Contracting States:
**BE SE**

(56) References cited:
**EP - A - 0 100 651**

**CHEMICAL ABSTRACTS, vol. 101, no. 19, 5th November 1984, page 39, column 1, abstract no. 172322b, Columbus, Ohio, US**
**CHEMICAL ABSTRACTS, vol. 85, no. 3, 3rd July 1976, page 638, column 1, abstract no. 20783r, Columbus, Ohio, US; L.P. ZALUKAEV et al. "Synthesis of isopropylidene p-alkylbenzylidenemalonates"**
**CHEMICAL ABSTRACTS, vol. 86, no. 4, 24th January 1977, page 30, column 1, abstract no. 17398j, Columbus, Ohio, US; C.P. PINAZZI et al. "Synthesis of new photocrosslinkable polymers derived from cinnamic acid"**
**BULLETIN OF THE CHEMICAL SOCIETY OF JAPAN, vol. 56, no. 11, 1983, pages 3517-3518, Tokyo, JP; H. KAMOGAWA et al. "Mesomorphic phenyl benzoate and 1,3-dioxane derivatives bearing terminal vinyl group"**

(73) Proprietor: **BEECHAM GROUP PLC, Beecham House Great West Road, Brentford Middlesex TW8 9BD (GB)**

(72) Inventor: **Buckle, Derek Richard, 18 Hillfield Road, Redhill Surrey (GB)**

(74) Representative: **Dayneswood, Trevor et al, Beecham Pharmaceuticals Patent and Trade Mark Department Biosciences Research Centre Great Burgh Yew Tree Bottom Road, Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to novel arachidonic acid analogues, to processes for preparing them, to pharmaceutical compositions containing them and their use in medicine.

It is known that certain arachidonic acid metabolites can produce harmful effects in man. For example, some prostaglandins and thromboxanes, produced via cyclooxygenation of arachidonic acid, can contribute to inflammation in such diseases as rheumatoid arthritis, and that products produced via lipoxygenation of arachidonic acid, such as the leukotrienes, are implicated in the production of the pathology of asthma and other allergic diseases.

European Published Patent Application No. 0109225 discloses certain arachidonic acid analogues which can inhibit arachidonic acid metabolism by one or both of these metabolic pathways.

We have now discovered a new class of arachidonic acid analogues which can similarly inhibit arachidonic acid metabolism and are thus of value in the prophylaxis and treatment of diseases whose symptoms are controlled by these mediators.

According to the present invention there is provided a compound of formula (I):

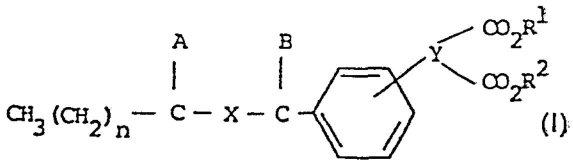

(I)

or a salt thereof,

in which Y is a group $-O(CH_2)_mCH<$,

$-(CH_2)_mCH<$, or $-CH=CH<$

where m is an integer of from 1 to 5
n is an integer of from 4 to 12
each of $R^1$ and $R^2$, which may be the same or different, represents hydrogen or $C_{1-6}$ alkyl

X represents a double or triple bond, and each of A and B represents hydrogen when X is a double bond, or both A and B are absent when X is a triple bond.

When X is a double bond, the hydrocarbon chain containing X may have the (E) or (Z) absolute configuration, preferably (Z).

Similarly, when Y is $-CH=C<$,

the 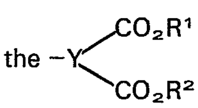 group

may have the (E) or (Z) absolute configuration.

The compounds of this invention can exist, therefore, in up to four geometric isomeric forms, and the invention encompasses all geometric isomers of the compounds of formula (I) whether as individual isomers or admixed with each other in any proportion.

The substituents on the aromatic ring may be in the 1,2; 1,3; or 1,4 positions, preferably in the 1,2 or 1,3 positions.

Salts of compounds of formula (I) need not be pharmaceutically acceptable, since they can be used as intermediates to prepare other pharmaceutically acceptable compounds of the invention.

Examples of pharmaceutically acceptable salts include alkali metal and alkaline earth metal salts, such as sodium, potassium and magnesium salts; and salts with ammonia, organic bases and amino compounds.

Preferably, n is an integer of from 8 to 12.

Preferably, m is 1, 2, or 3.

According to a further aspect of the invention there is provided a process for preparing a compound of the invention which comprises treating a compound of formula (II).

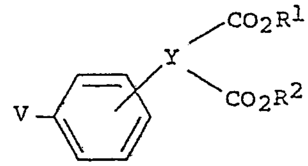

(II)

in which Y is as defined with reference to formula (I), each of $R^1$ and $R^2$ are $C_{1-6}$ alkyl,

V represents 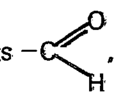, $-\overset{\ominus}{C}H-\overset{\oplus}{P}Ph_3$ or halogen,

preferably bromine or iodine, with a compound of formula (III),

$$CH_3(CH_2)_n-W \qquad (III)$$

in which W is 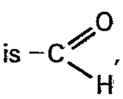, $-\overset{\ominus}{C}H-\overset{\oplus}{P}Ph_3$, $-CH=CH_2$

or $-C\equiv CH$,

and optionally thereafter reducing the carbon-carbon triple bond, when present in the resultant product, to a carbon-carbon double bond, and/or optionally converting the resultant product to the corresponding mono- or dibasic acid with provisos that

i) when V is 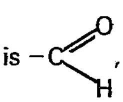, W is $-\overset{\ominus}{C}H-\overset{\oplus}{P}Ph_3$,

ii) when V is $-\overset{\ominus}{C}H-\overset{\oplus}{P}Ph_3$, W is 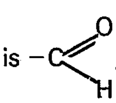, and

iii) when V is halogen, W is $-CH=CH_2$ or $-CH\equiv CH$

When V is halogen, preferably bromine or iodine and W is $-CH=CH_2$ or $C\equiv CH$, the reaction is preferably carried out by refluxing the reactants in the presence of a palladium (II) salt/triarylphosphine catalyst in a tertiary amine, such as triethylamine, as a diluent and proton sink. A preferred catalyst is palladium acetate/triphenylphosphine, $Pd(OAc)_2[Ph_3P]_2$.

The resultant compound of the invention may be separated from the reaction mixture by chromatography of the filtered and evaporated reaction mixture.

When V or W is 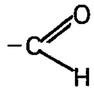 or $-\overset{\ominus}{C}H-\overset{\oplus}{P}Ph_3$, the reaction is preferably carried out in a suitable solvent, preferably dimethylsulphoxide or tetrahydrofuran, at ambient temperature and the product purified chromatographically. The reaction will generally produce a mixture of geometrical isomers which can be separated in conventional manner, such as by chromatography, for example on argentated silica gel.

When the resultant compound of the invention formed by reacting compounds of formula (II) and (III) includes a carbon-carbon triple bond, as will occur when W is $-C\equiv CH$, a further compound of the invention may be formed by reducing the triple bond to a double bond. This reduction may be carried out by conventional literature procedures, preferably by hydrogenation in the presence of a Lindlar catalyst, or other poisoned catalyst such as palladium on barium sulphate. This reduction tends to be stereospecific to the extent that the $CH_3(CH_2)_n-W-$ chain in the resultant compound of the invention has the (Z) configuration.

The resulting compound of the invention in which each of $R^1$ and $R^2$ is $C_{1-6}$ alkyl may be quantitatively hydrolised with base, for example lithium hydroxide in aqueous tetrahydrofuran, to give a mono- or dibasic acid of formula (I), in which only one of $R^1$ and $R^2$ is hydrogen, or both of $R^1$ and $R^2$ are hydrogen, respectively. In general, treatment with 1 mole equivalent of base will yield a monobasic acid, and 2 mole equivalents will yield a dibasic acid. When Y in formula (I) is $-CH=C<$, the monobasic acids of formula (I) may have different geometric isomerism, according to whether $R^1$ or $R^2$ is hydrogen.

The intermediate compounds of formula (II) may be prepared in a number of ways, as described hereinafter.

Compounds of formula (II) in which Y is $-O(CH_2)_mCH<$ and V is 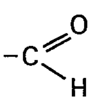 may be prepared by treating an hydroxy benzaldehyde with a bromo-alkanoate ester of formula (IIA)

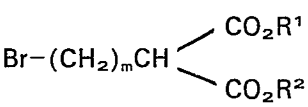 (IIA)

wherein m is as defined in formula (I) and $R^1$ and $R^2$ are each $C_{1-6}$ alkyl, according to the general method disclosed in British Patent Specification No. 1350883.

Compounds of formula (II) in which Y is $-O(CH_2)_mCH<$ and V is $-\overset{\ominus}{C}H-\overset{\oplus}{P}Ph_3$ may be prepared by treating a compound of formula (IIB).

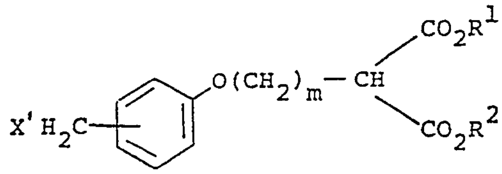 (IIB)

in which m is as defined in formula (I), and $R^1$ and $R^2$ are each $C_{1-6}$ alkyl, and X′ is halogen, preferably bromine, with triphenylphosphine to form the phosphonium salt, followed by proton abstraction with a strong base such as n-butyl lithium.

Compounds of formula (IIB) may themselves be prepared by reducing a compound of formula (II) in which Y is $-O(CH_2)_mCH<$ and V is 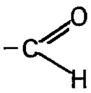 to the corresponding alcohol, and subsequently treating the alcohol with a halogenating agent. The reduction is conveniently carried out by sodium borohydride, and a preferred halogenating agent is carbon tetrabromide triphenyl phosphine.

Compounds of formula (II) in which Y is $-CH=C<$ and V is $-\overset{\ominus}{C}H-\overset{\oplus}{P}Ph_3$ may be prepared by reacting a compound of formula (IID).

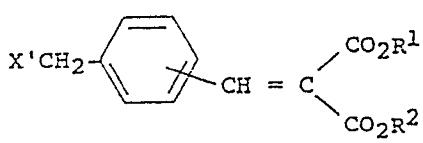 (IID)

wherein X′ is halogen, preferably bromine, with triphenylphosphine, and subsequently treating the resulting compound of formula (IIE).

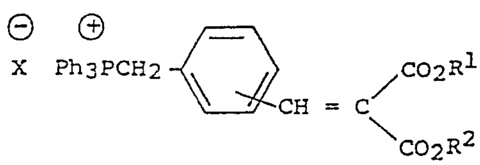 (IIE)

with sodium methylsulphinylmethylide in dimethylsulphoxide.

Compounds of formula (IID) may themselves be prepared in an analogous manner to those of formula (IIB).

Compounds of formula (II) in which V is halogen and Y and R are as defined in formula (II) are either known compounds or can be prepared from known compounds by knwon methods. For example, a bromobenzaldehyde may be condensed with a dialkyl malonate under standard Knoevenagel conditions (M.S. Newman and D.K. Phillips *J. Amer. Chem. Soc. 81*, 3667 (1959)) to give a compound of formula (II) in which V is bromine and

Y is $-CH=C<$

Also, a dibromide of formula (IIF)

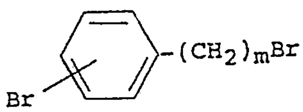 (IIF)

may be coupled with a dialkyl malonate under standard base mediated conditions (F.G. Holliman and F.G. Mann *J. Chem. Soc. 9*, (1960)) to give a compound of formula (II) in which V is bromine and

Y is $-(CH_2)_mCH<$,

in which m is as defined in formula (I).

Compounds of formula (II) in

which Y is $-CH=C<$ and V is 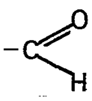

can be prepared from known compounds by known methods.

The intermediate compounds of formula (III) in

which W is 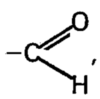, $-CH=CH_2$ or $-C\equiv CH$ are

either known compounds or can be prepared from known compounds by known methods.

The compounds of formula (III) in which W is $-\overset{\ominus}{C}H-\overset{\oplus}{P}Ph_3$ may be prepared by reacting the corresponding phosphonium bromide with sodium methylsulphinylmethylide, according to the method described in *J. Org. Chem., 28*, 1128, 1963 (Greinwald et al), or by reaction with butyl lithium in tetrahydrofuran.

Compounds of the invention may also be prepared by alternative processes, described as follows.

Compounds of formula (I) in which $R^1$ and $R^2$ are each $C_{1-6}$ alkyl and Y is

$-O(CH_2)_mCH<$ or $-(CH_2)_mCH<$

may be prepared by treating a compound of formula (IV),

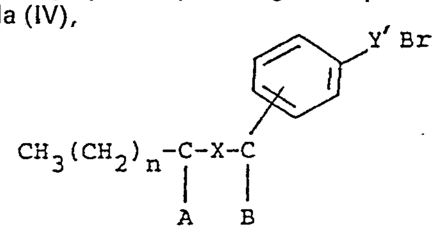 (IV)

in which n, A, B and X are as defined in formula (I), and Y' is $-O(CH_2)_m-$ or $-(CH_2)_m-$, with a malonic diester of formula (V)

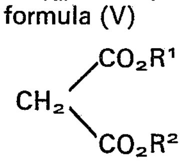 (V)

in which $R^1$ and $R^2$ are each $C_{1-6}$ alkyl.

Compounds of formula (IV) may themselves be prepared by (a) treating a compound of formula (IVA).

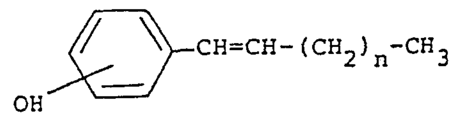 (IVA)

with a compound of formula (IVB).

$Br(CH_2)_mBr$ (IVB)

wherein m and n are as defined in formula (I), or (b) treating a compound of formula (IVC)

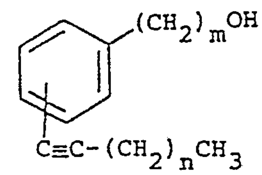 (IVC)

with a mixture of triphenylphosphine and carbon tetrabromide, preferably in dichloromethane.

The reaction between compounds of formulae (IVA) and (IVB) is preferably carried out in a basic medium such as potassium carbonate in butanone.

Compounds of formula (IVA) can themselves be prepared by treating an hydroxy benzaldehyde with a compound of formula (III), in which W is $-\overset{\ominus}{C}H-\overset{\oplus}{P}Ph_3$, preferably in a solvent such as dimethylsulphoxide or tetrahydrofuran at ambient temperature.

Compounds of formula (IVC) can be prepared by reducing a compound of formula (VI)

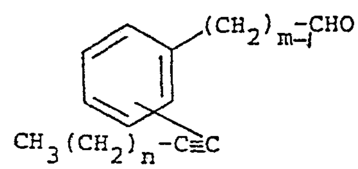 (VI)

preferably with sodium borohydride or diisobutylaluminium hydride (DIBAL).

Compounds of formula (I) in which $R^1$ and $R^2$ are each $C_{1-6}$ alkyl, Y is

$-CH=C<$

and X is a triple bond may be prepared by treating a compound of formula (VI) as defined above, wherein m = 1, with a compound of formula (V) as defined above.

The reaction is preferably carried out by refluxing the reactants in benzene or toluene as a diluent, and a catalyst such as piperidine/benzoic acid such that the water produced is removed azeotropically.

The compounds of formula (VI) may themselves be prepared by treating a compound of formula (VII).

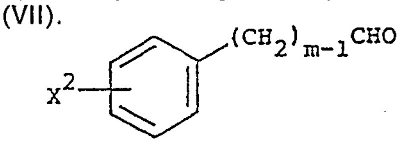 (VII)

in which $X^2$ is bromine or iodide, preferably bromine, with a compound of formula (III) in which W is $-C\equiv CH$.

The reaction is preferably carried out by refluxing the reactants in the presence of a palladium (II)

salt/triarylphosphine catalyst, as described hereinbefore.

Compounds of formulae (V) and (VII) are either known compounds or can be prepared from known compounds by known methods.

Compounds of formula (I) in which $R^1$ and $R^2$ are each $C_{1-6}$ alkyl and Y is

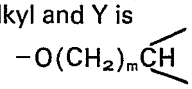

may also be prepared by treating a compound of formula (VIII)

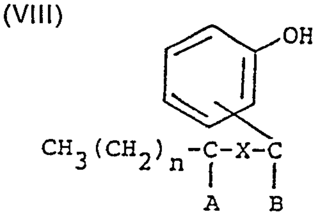

(VIII)

in which n, A, B and X are as defined in formula (I) with a compound of formula (IX)

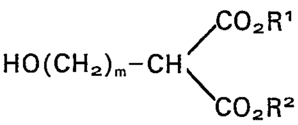

(IX)

in which m, $R^1$ and $R^2$ are as defined in formula (I).

The reaction is preferably carried out in an inert organic solvent such as tetrahydrofuran, in the presence of triphenylphosphine and diethyl azodicarboxylate.

Compounds of formula (VIII) in which X is a triple bond may be prepared using the following reaction schemes

(a)

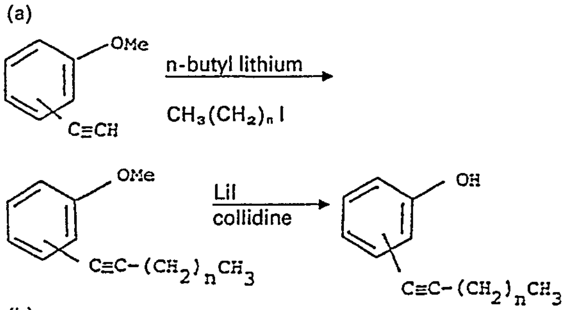


(b)

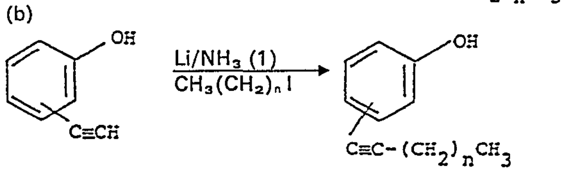


Reaction scheme (a) is, however, not suitable for those cases in which the generated hydroxyl groups and the appended chain are ortho to each other.

The compounds of formula (I) are active therapeutically, and, accordingly, this invention also provides a pharmaceutical composition comprising a compound of formula (I) as defined above, or a pharmaceutically acceptable salt thereof, and a pharmaceutically acceptable carrier.

A compound of formula (I), or salt thereof, which is active when given by the oral route, may be compounded in the form of syrups, tablets, capsules, pills and the like. Preferably, the composition is in unit dosage form, or in a form in which the patient can administer to himself a single dosage. When the composition is in the form of a tablet, powder or lozenge, any pharmaceutical carrier suitable for formulating solid compositions may be used. Examples of such carriers are magnesium stearate, starch, lactose, glucose, sucrose, rice flour and chalk. The composition may also be in the form of an ingestible capsule (e.g. of gelatin) containing the compound; or in the form of a syrup, a liquid solution or a suspension. Suitable liquid pharmaceutical carriers include ethyl alcohol, glycerine, saline and water which may be compounded with flavouring or colouring agents to form syrups.

The compounds of this invention may also be administered by a non-oral route. In accordance with routine pharmaceutical procedure, the compositions may be formulated, for example for rectal administration as a suppository or for presentation in an injectable form in an aqueous or nonaqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers solutes to render the solution isotonic with the blood, thickening agents, suspending agents or other pharmaceutically acceptable additives. Such forms will be presented in unit dose form such as ampoules or disposable injection devices or in multidose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts of other antiasthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

A compound of general formula (I) or salt thereof, may also be presented as an ointment, cream, lotion, gel, aerosol, or skin paint for topical application.

It is preferred that the compounds of this invention are administered by inhalation.

By way of example, in any of the preceding formulations a suitable dosage unit might contain 0.01 to 500mgs of active ingredient, more suitably 1 to 500mgs *via* the oral route, 0.01 to 10mgs *via* inhalation. The effective dose of compound depends on the particular compound employed, the condition of the patient and on the frequency and route of administration, but in general is in the

range of from 0.001 mg/kg/day to 100 mg/kg/day inclusive of the patient's body weight.

Within the above indicated dosage range, no adverse toxicological effects have been observed with the compounds of the invention.

As in common practice, the compositions will usually be accompanied by written or printed directions for use in the medical treatment concerned, in this case as an anti-allergic agent for the prophylaxis and treatment of, for example, asthma, hay fever, rhinitis or allergic eczaema.

The invention also provides a method for treating allergic diseases in human and non-human animals, which comprises administering to the sufferer an effective non toxic amount of a compound of formula (I) or a pharmaceutically acceptable salt thereof.

The following Examples illustrate the preparation of compounds of this invention.

*Example 1:*

*Diethyl 2-(2-bromophenyl)methylene propan-1,3-dioate*

A solution of 2-bromobenzaldehyde (45g) and diethyl malonate (40g) in dry benzene (125ml) was treated with benzoic acid (1.5g) and piperidine (1.75ml) and refluxed for 17hours under a Dean and Stark head. A further 0.5ml of piperidine was then added and the mixture refluxed for a further 7 hours and then cooled. Ether was added and the organic phase was washed with dilute hydrochloric acid, sodium bicarbonate solution and then dried. Evaporation and distillation gave 55.22g (69%) of pale yellow oil of $bp_{0.2}$ 162-164° C (lit $bp_{1.0}$ 178-181° C, M.S. Newman and D.K. Phillips, *J. Amer. Chem. Soc.*, *81*, 3667, [1959]).

*Example 2:*

*Diethyl 2-(4-bromophenyl)methylene propan-1,3-dioate*

The method used was that described in example 1 to give the title compound in 86% yield of $bp_{0.5}$ 147-152° C, mp 43-44° C.

Found: C, 51.50, H, 4.38, $C_{14}H_{15}BrO_4$
Requires: C, 51.39, H, 4.62%

*Example 3:*

*2-(1-Tridecynyl) benzaldehyde*

Palladium acetate (96mg) was added to a deaerated mixture of 2-bromobenzaldehyde (7.40g, 40mmol), 1-tridecyne (12.2g, 68mmol) and triphenylphosphine (320mg) in dry triethylamine (160ml) and the mixture was stirred at 105° C under a nitrogen atmosphere overnight. After cooling, the precipitated triethylamine hydrobromide was filtered off and the filtrate was evaporated to a crude red oil. Chromatography on silica gel eluting with dichloromethane: petroleum ether [bp 60-80° C] (1:1) afforded 7.12g (63%) of the title compound of $bp_{0.05}$ 212° C (Kugelrohr), $M^+$ 284.2141 ($C_{20}H_{28}O$ requires; 284.2140).

Found: C, 84.31, H, 10.30, $C_{20}H_{28}O$
Requires: C, 84.45, H, 9.92%

The 2,4-dinitrophenylhydrazone had mp (acetic acid) 132° C. Found: C, 67.17, H, 6.84, N, 11.98, $C_{26}H_{32}N_4O_2$. Requires: C, 67.22, H, 6.94, N, 12.06%.

*Example 4:*

*4-(1-Octynyl) benzaldehyde*

The method as described in example 3 was followed to give the title compound in 52% yield after chromatography on silica eluting with dichloromethane:hexane (1:3). The 2,4-dinitrophenylhydrazone had mp (EtOH) 180-183° C. Found: C, 63.31, H, 5.71, N, 13.06, $C_{21}H_{22}N_4O_4 \cdot 0.5C_2H_5OH$. Requires: C, 63.29, H, 6.03, N, 13.42%.

*Example 5:*

*3-(1-Tridecynyl) benzaldehyde*

Reaction of 3-bromobenzaldehyde with 1-tridecyne as described in example 3 gave 85% of the title compound after chromatography on silica, eluting with dichloromethane: petrol (bp 40:60) (1:3) $v_{max}$ (neat) 2920, 2215, 1705, 1600, 1575, 1465, 1280, 1155, 795, and 680cm$^{-1}$, δ ($CDCl_3$) 0.86 (3H, distorted t), 0.9-1.7 (18H, m), 2.4 (2H, t, J = 6Hz), 7.7-7.9 (4H, m, aromatic), and 10.0 (1H, s, aldehyde). Analysed as the 2,4-dinitrophenylhydrazone m.p. 127° C (recrystallized from acetic acid). Found: C, 67.27, H, 6.86, N, 12.01, $C_{26}H_{32}N_4O_4$. Requires: C, 67.33, H, 6.79, N, 12.08%.

*Example 6:*

*Diethyl 2-[(1-tridecynyl)phenyl]methylenepropan-1,3-dioate*

Reaction of diethyl 2-(2-bromophenyl)-methylenepropan-1,3-dioate (6.54g, 20mmole, from example 1) with 1-tridecyne (6.1g, 34mmole) as described in example 3 gave 5.37g (63%) of the title compound after chromatography on silica gel eluting with dichloromethane: petroleum ether [bp 60-80° C] (1:1). It had, $v_{max}$ (film) 1730, 2850, 2920cm$^{-1}$, δ ($CDCl_3$) 0.84 (3H, distorted t, terminal $CH_3$), 1.13-1.68 (24H, m, ester $CH_3$ + alkylene chain), 2.42 (2H, t, J 6.3Hz, $\equiv-CH_2$), 4.23 (2H, q, J 7.5Hz, ester $CH_2$), 4.26 (2H, q, J 7.5Hz, ester $CH_2$), 7.31 (4H, m, aromatics), 8.20 (1H, s, CHO), $M^+$ 426.2780 ($C_{27}H_{38}O_4$ requires; 426.2770).

*Example 7:*

*Diethyl 2-[2-(1-tridecynyl)phenyl]methylene-propan-1,3-dioate*

A solution of 2-(1-tridecynyl) benzaldehyde (6.42, 22.6mmol from example 3) was condensed with diethyl malonate (3.72g) as described in example 1 to give 2.00g (21%, 36% on the basis of unrecovered starting material) of the title compound identical with that prepared in example 6 above.

*Example 8:*

*Diethyl 2-[4-(1-tridecynyl)phenyl] methylene-propan-1,3-dioate*

The method followed was that described in example 3 to give the title compound in 35% yield, as an oil after chromatography.

Found: C, 76.10, H, 9.20, $C_{27}H_{38}O_4$
Requires: C, 76.02, H, 8.98%

*Example 9:*

*Diethyl 2-[4-(1-octynyl)phenyl] methylene-propan-1,3-dioate*

The method described in example 3 was followed to give the title compound as an oil in 32% yield after chromatography.

$M^+$ 356.1990 ($C_{22}H_{28}O_4$ requires 356.1988).

*Example 10:*

*Diethyl 2-(2-[(Z)-1-tridecenyl]phenyl) methyl-enepropan-1,3-dioate*

5% Palladium on barium sulphate (64mg) was added to a solution of diethyl 2-[2-(1-tridecynyl)phenyl] methylenepropan-1,3-dioate (1.28g, 3mmol, from example 6) in pyridine (20ml) and the mixture was hydrogenated at atmospheric pressure until 1 equivalent of hydrogen was absorbed. The catalyst was removed by filtration and the pyridine evaporated *in vacuo* to leave an oil. Chromatography on 10% argentated silica gel eluting with dichloromethane: petroleum ether [bp 60-80° C] (3:2) gave 0.91g (71%) of the Z-alkene as a pale yellow oil, $v_{max}$ 1065, 1205, 1250, 1630, 1730, 2850, 2920cm$^{-1}$; δ ($CDCl_3$) 0.84 (3H, distorted t, terminal $CH_3$), 2.22 (24H, m, alkylene chain + ester $CH_3$), 2.00 (2H, m, allylic $CH_2$), 4.16 (2H, q, J *ca* 7.5Hz, ester $CH_2$), 4.23 (2H, q, J *ca* 7.5Hz, ester $Ch_2$), 5.77 (1H, d.t, $J_d$ 11.5Hz, $J_t$ 7.5Hz, C=CH-alkyl), 6.41 (1H, d, J 11.5Hz, ArCH=C-alkyl), 7.22 (4H, m, aromatics), 7.87 (1H, s, ArCH=C($CO_2R)_2$). $M^+$ 428.2924 ($C_{27}H_{40}O_4$ requires, 428.2926).

*Example 11:*

*Diethyl 2-(4-[(Z)-1-tridecenyl]phenyl) methyl-enepropan-1,3-dioate*

The method described in example 10 was followed to give the title compound as an oil in 64% yield after chromatography.

Found: C, 75.82; H, 9.64; $C_{27}H_{40}O_4$
Requires: C, 75.66; H, 9.41%

*Example 12:*

*Diethyl 2-(2-bromobenzyl) propan-1,3-dioate*

Diethyl malonate (77g, 0.48mol) was added to a stirred solution of sodium (10.2g, 0.425mol) in ethanol (800ml) followed by 2-bromobenzyl bromide (100g, 0.40mol). Sodium bromide began to separate towards the end of the addition and the mixture became warm. The reaction was completed by refluxing on a steam bath for 3 hours and then cooling overnight. The bulk of the solvent was removed *in vacuo*, water was added and the product extracted into ether. After drying ($CaCl_2$) and evaporation the product was distilled to give 93.28g (71%) of material of $bp_{0.2}$ 130° C (lit $bp_{0.004}$ 115-120° C, F.G. Holliman and F.G. Mann *J. Chem. Soc.* 9, [1960]).

*Example 13:*

*Diethyl 2-(4-bromobenzyl)propan-1,3-dioate*

The method described in example 12 was followed to give the title compound in 50% yield as an oil ($b_{0.5}$ 133-136° C).

Found: C, 51.21, H, 5.26, $C_{14}H_{19}O_4$
Requires: C, 51.08, H, 5.21%

*Example 14:*

*Diethyl 2-[2-(1-tridecynyl)phenyl]methylpropan-1,3-dioate*

Reaction of diethyl (2-bromobenzyl) propan-1,3-dioate (11.87g, 30mmol) with 1-tridecyne (11.10g, 45mmol) as described in example 3 gave 3.89g (29%) of the title compound as a pale yellow oil after chromatography. It had $v_{max}$ (film) 1735, 1755, 2840, 2920cm$^{-1}$; δ ($CDCl_3$) 0.87 (3H, distorted t, terminal $CH_3$), 1.08 (6H, t, J 7Hz, ester $CH_3$), 1.28 (18H, m, alkylene chain), 2.42 (2H, t, J 6Hz, C=C−$CH_2$), 3.31 (2H, d, J 7.5Hz, Ar$CH_2$), 3.90 (1H, t, J 7.5Hz, methine H), 4.12 (4H, q, J 7.5Hz, ester $CH_2$), 7.15 (3H, m, aromatics), 7.36 (1H, m, aromatic). $M^+$ 428.2928 ($C_{27}H_{40}O_4$ requires; 428.2926).

*Example 15:*

*4-[(Z)-1-Undecenyl] phenol*

Sodium hydride (1.68g, 0.035mol of a 50% dispersion in mineral oil) was washed by decantation with petroleum ether bp 60-80° C and added to dry deaerated dimethylsulphoxide (40ml) under nitrogen. The mixture was stirred at 75° C for 0.5h, cooled to 5° C in an ice bath and a solution of n-decyltriphenylphosphonium bromide (16.78g, 0.035M) in dimethylsulphoxide (50ml), added. The red solution was stirred for 15min. and then treated dropwise with 4-hydroxybenzaldehyde, sodium salt (5.10g, 0.035M) in dry deaerated dimethylsulphoxide (30ml) at 10° C. After stirring at room temperature for 3h, the mixture was poured into water, acidified with dilute hydrochloric acid, extracted into ethyl acetate and dried ($MgSO_4$). Evaporation of the solvent gave a red oil which was purified by column chromatography on silica gel, eluting with dichloromethane to yield 6.15g (70%) of E/Z isomers as a white, crystalline solid. Recrystallization from petroleum ether bp 60-80° C gave 4.25g (50%) of the pure Z-isomer. A second crop was also obtained as a 65:35 mixture of Z and E isomers respectively (1.0g, 12% yield).

*Data 4-[(Z)1-undecenyl]phenol*

Mp (petroleum ether, bp 60-80° C) 57° C $v_{max}$ (mull) 1515, 1595, 1610, 1900 (weak) 3325cm$^{-1}$; δ ($CDCl_3$) 0.84 (3H, distorted t, terminal $CH_3$), 1.24 (14H, m, $(CH_2)_7$), 2.23 (2H, bt,

J 6Hz, allylic $CH_2$), 4.67 (1H, s, OH), 5.53 (1H, d.t, $J_d$ 12Hz, $J_t$ 7Hz, C=CH-alkyl), 6.30 (1H, d, J 12Hz, ArCH=C), 6.96 (4H, ABq, Δv 36Hz, J 9Hz, $C_6H_4$).

Found: C, 82.63, H, 10.54, $C_{17}H_{26}O$
Requires: C, 82.87, H, 10.64%

*Examples 16 and 17:*

*2-[(Z)1-Tridecenyl]phenol (Example 16) and 2-[(E)-1-Tridecenyl]phenol (Example 17)*

Prepared according to the method described in example 15 from salicyladehyde and dodecyltriphenylphosphium bromide. Chromatography of the crude reaction mixture on silica gel (dichloromethane) gave 4.48g of mixed E and Z isomers (44%). Rechromatography on silica gel (dichloromethane-hexane, 1:4) gave first 1.84g pure Z-isomer as a colourless oil (18%), $v_{max}$ (film) 3400, 1605, 1595, 1480cm$^{-1}$; δ ($CDCl_3$) 0.85 (3H, distorted t, terminal $CH_3$), 1.25 (18H, m, $(CH_2)_9$), 2.12 (2H, m, allylic $CH_2$), 5.00 (1H, bs, OH), 5.83 (1H, d.t, $J_d$ 12Hz, $J_t$ 7Hz, C=CH-alkyl), 6.32 (1H, d, J 12Hz, ArCH=C), 7.00 (4H, m, aromatic).

Found: C, 81.86, H, 10.77, $C_{19}H_{30}O$, 0.25$H_2O$
Requires: C, 81.80, H, 11.02%

M.S. Observed mass 274.2298, Theoretical mass 274.2297.

Further evaluation gave the (E)-isomer (1.97g) as a colourless oil (19%), $v_{max}$ (film) 3350, 1605, 1590, 1480cm$^{-1}$; δ ($CDCl_3$) 0.90 (3H, distorted t, terminal $CH_3$), 1.28 (18H, m, $(CH_2)_9$), 2.14 (2H, m, allylic $CH_2$), 5.15 (1H, bs, OH), 6.10 (1H, d.t, $J_d$ 16Hz, $J_t$ 8Hz, C=CH alkyl), 6.48 (1H, d, J 16Hz), 7.05 (4H, m, aromatic).

M.S. Observed mass 274.2304, Theoretical mass 274.2297.

*Example 18:*

*1-Bromo-2-[4-(Z)-1-Undecenyl]phenoxy) ethane*

4-[(Z)-Undecenyl] phenol (2.30g, 9.30mmol) and anhydrous potassium carbonate (1.93g, 14.0mmol) in methyl isobutyl ketone (100ml) were stirred and treated with 1,2-dibromoethane (5.27g, 29mmol). The mixture was refluxed for 24h after which time a further aliquot of 1,2-dibromoethane (5.27g, 28mmol) was added. The mixture was refluxed a further 24h, then cooled and washed with water (twice). The organic phase was dried ($MgSO_4$), and evaporated *in vacuo* to an oil. Purification by column chromatography on silica gel, eluting with n-hexane then dichloromethane gave 1.34g (41%) of the title compound as a white crystalline solid mp 40-1° C. $v_{max}$ (mull) 1510, 1573 (weak) 1610cm$^{-1}$; δ ($CDCl_3$) 0.86 (3H, distorted t, terminal $CH_3$), 1.26 (14H, m, $(CH_2)_7$), 2.26 (2H, m, allylic $CH_2$), 3.60 (2H, t, J 6Hz, $CH_2Br$), 4.26 (2H, t, J 6Hz, $OCH_2$), 5.56 (1H, d.t, $J_d$ 12Hz, $J_t$ 7 Hz, =CH-alkyl), 6.32 (1H, d, J 12Hz, ArCH=), 7.06 (4H, ABq, Δv 33Hz, J 9Hz, $C_6H_4$).

Found: C, 64.55, H, 8.43, Br, 22.33, $C_{19}H_{29}BrO$
Requires: C, 64.58, H, 8.27, Br, 22.62%

Mass Spec. Observed mass 352.1403, Theoretical mass 352.1402 for $C_{19}H_{29}BrO$.

*Example 19:*

*1-Bromo-2-(2-[(Z)1-tridecenyl] phenoxy) ethane*

Prepared from 2-[(Z)-1-tridecenyl] phenol 1.70g (example 16) by the method described in example 18.

After chromatography of the crude reaction mixture the title compound was isolated (1.23g = 52%). Some unreacted phenol (0.42g) was also isolated. (Yield based on phenol consumed 69%) $v_{max}$ 1600, 1450, 1240, 745cm$^{-1}$; δ ($CDCl_3$) 0.92 (3H, distorted t, terminal $CH_3$), 1.28 (18H, m, $(CH_2)_9$), 2.22 (2H, m, allylic $CH_2$), 3.57 (2H, t, $CH_2Br$), 4.22 (2H, t, $OCH_2$), 5.65 (1H, d.t, $J_d$ 12Hz, $J_t$ 7Hz, C=CH alkyl), 6.58 (1H, d, ArCH=C), 7.05 (4H, m, aromatic).

Found: C, 66.22, H, 8.80, Br, 20.66%, $C_{21}H_{33}BrO$
Requires: C, 66.12, H, 8.72, Br, 20.95%

M.S. Observed mass 380.1719, Theoretical mass 380.1715.

*Example 20:*

*1-Bromo-2-(-2-[(E)-1-tridecenyl] phenoxy) ethane*

Prepared from 2-[(E)-1-tridecenyl] phenol 1.05g (Example 17) by the method described in Example 18.

Chromatography on silica gel (Dichloromethane-hexane 1:4) gave the product (0.93g = 64%) as a pale yellow oil, $v_{max}$ 1600, 1490, 1455, 1240, 745cm$^{-1}$; δ ($CDCl_3$) 0.95 (3H, distorted t, terminal $CH_3$), 1.30 (18H, m, $(CH_2)_9$), 2.22 (2H, m, allylic $CH_2$), 3.63 (3H, t, $CH_2Br$), 4.25 (3H, t, $OCH_2$), 6.22 (1H, d.t, $J_d$ 16Hz, $J_t$ 8Hz, C=CH alkyl), 6.62 (1H, d, J 16Hz, ArCH=C), 7.10 (4H, m, aromatic).

Found: C, 65.95, H, 8.77, Br, 21.11%, $C_{21}H_{33}BrO$
Requires: C, 66.12, H, 8.72, Br, 20.95%

M.S. Observed mass 380.1705, Theoretical mass 380.1715.

*Example 21:*

*Diethyl 2-(4-[(Z)-1-undecenyl]phenoxyethyl) propan-1,3-dioate*

A solution of sodium (0.408g, 17.7mmol) in ethanol (50ml) under nitrogen was treated with diethyl malonate (2.84g, 17.7mmol) followed by 1-bromo-2-(4-[(Z)1-undecenyl]phenoxy) ethane (1.30g, 3.68mmol). The solution was refluxed 30h, then cooled and evaporated *in vacuo* to dryness. The residue was partitioned between ethyl acetate and water and the organic phase separated, washed with water, and dried ($MgSO_4$). Evaporation *in vacuo* gave 2.0g of a yellow oil which was purified by column chromatography using silica gel, eluting with dichloromethane: n-hexane [2:3] to yield 510mg (32%) of the title compound as a colourless oil. $v_{max}$ (film) 1510, 1570 (weak), 1608, 1735, 1753cm$^{-1}$; δ ($CDCl_3$) 0.84 (3H, distorted t, terminal $CH_3$), 1.25 (3H, t, J 7Hz, ester $CH_3$), 1.25 (14H, m, $(CH_2)_7$), 2.31 (4H, m, $OCH_2\underline{CH_2}$, allylic $CH_2$), 3.63 (1H, t, J 7Hz, $\underline{CH}(CO_2Et)_2$), 4.01 (2H, t, J 7Hz, $OCH_2$), 4.18 (4H, q, J 7Hz, ester $CH_2$), 5.54 (1H, d.t, $J_d$ 12Hz, $J_t$ 7Hz, =CH-alkyl), 6.31 (1H, d, J 12Hz, ArCH=), 7.02 (4H, ABq, Δv 34Hz, J 9Hz, $C_6H_4$).

Mass Spec. Observed mass 432.2887, Theoretical mass 432.2876 for $C_{26}H_{40}O_5$.

Found: C, 72.15, H, 9.49, $C_{26}H_{40}O_5$
Requires: C, 72.19, H, 9.32%

*Example 22:*

*Diethyl 2-(2-[(z)-1-tridecenyl]phenoxyethyl)-propan-1,3-dioate*

Prepared from the bromoethyl compound (1.20g) (Example 19) and diethylmalonate by the method described in Example 21. Chromatography of the crude product on silica gel (dichloromethane-hexane 3:7) gave the product 0.81g (60%) as a colourless oil. $v_{max}$ 1750, 1730, 1600, 1450, 1240, 745cm$^{-1}$; δ ($CDCl_3$) 0.90 (3H, distorted t, terminal $CH_3$), 1.28 (24H, m, $(CH_2)_9$+2×ester $CH_3$), 2.35 (4H, m, allylic $CH_2$+$\underline{CH_2}CH(CO_2Et)_2$), 3.70 (1H, t, $CH_2\underline{CH}(CO_2Et)_2$), 4.22 (6H, m, $OCH_2$+2×ester $CH_2$), 5.65 (1H, d.t, $J_d$ 12Hz, $J_t$ 7Hz, C=CH alkyl), 6.48 (1H, d, J 12Hz, ArCH=C), 7.00 (4H, m, aromatic).

Found: C, 72.75, H, 9.46%, $C_{28}H_{44}O_5$
Requires: C, 73.00, H, 9.63%

M.S. Observed mass 460.3188, Theoretical mass 460.3189.

*Example 23:*

*Diethyl 2-(2-[(E)-1-tridecenyl] phenoxyethyl) propan-1,3-dioate*

Prepared from the bromoethyl compound (0.70g) (Example 20) and diethylmalonate by the method described in Example 21. Chromatography of the crude product on silica gel (dichloromethane-hexane 1:4) gave the product 0.42g (50%) as a colourless oil, $v_{max}$ 1755, 1735, 1600, 1240, 745cm$^{-1}$; δ ($CDCl_3$) 0.92 (3H, distorted t, terminal $CH_3$), 1.30 (24H, m, $(CH_2)_9$+2×ester $CH_3$), 2.23 (2H, m, allylic $CH_2$), 2.42 (2H, q, $\underline{CH_2}CH(CO_2Et_2)$), 3.65 (1H, t, $CH_2\underline{CH}(CO_2Et)_2$), 4.15 (6H, m, $OCH_2$+2×ester $CH_2$), 6.17 (1H, d.t, $J_d$ 16Hz, $J_t$ 8Hz, C=CH alkyl), 6.58 (1H, d, J 16Hz, ArCH=C), 7.00 (4H, m, aromatic).

Found: C, 73.05, H, 9.73%, $C_{28}H_{44}O_5$
Requires: C, 73.00, H, 9.63%

M.S. Observed mass 460.3197, Theoretical mass 460.3189.

*Examples 24-33 (Tables I and II)*

*General hydrolysis procedures*

a) *Mono acids* – A solution of the diester (10mmol) in tetrahydrofuran (20ml) was added to a solution of lithium hydroxide monohydrate (420mg, 10mmol) in water (10ml) and the mixture was stirred at 30→60° C until neutral (⩽ 24hrs). The mixture was then cooled and acidified with dilute hydrochloric acid and the product extracted into ether. The ethereal phase was dried ($MgSO_4$) and evaporated and the residue either recrystallized or chromatographed on silica gel to give pure mono acids. Small samples of the diacids were sometimes isolated by this procedure also.

b) *Diacids*– A mixture of the diester (3mmol) in water (20ml) containing potassium hydroxide (1.8g) was stirred at 100° C for 6→24hrs and the resulting clear solution was cooled, added to an equal volume of water and evaporated to one half its bulk, *in vacuo.* Acidification then yielded the product which was either filtered off and recrystallized or isolated by ethereal extraction.

*Table I*

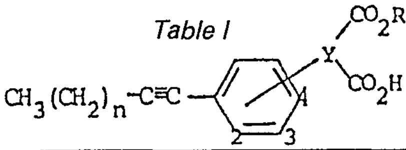

| Ex. No. | n | Y | Position of Y attachment | R | Yield % | mp, °C | Recrystallization Solvent | Formula | Analysis (calcd/found) C | H |
|---|---|---|---|---|---|---|---|---|---|---|
| 24 | 10 | CH=C | 2 | Et | 66 | 48-50 | petroleum ether bp 60-80°C | $C_{25}H_{34}O_4$ | 75.34<br>75.36 | 8.60<br>8.69 |
| 25 | 10 | CH=C | 2 | H | 79 | 93-96 | petroleum ether bp 80-100°C | $C_{23}H_{30}O_4 \cdot 0.5H_2O$ | 72.79<br>72.78 | 8.23<br>8.31 |
| 26 | 10 | CH=C | 4 | Et | 64 | 94-95 | hexane | $C_{25}H_{34}O_4$ | 75.34<br>75.17 | 8.59<br>8.49 |
| 27 | 10 | CH=C | 4 | H | 59 | 99-100 | hexane | $C_{23}H_{30}O_4$ | 75.34<br>75.16 | 8.60<br>8.71 |
| 28 | 10 | $CH_2CH_2$ | 2 | H | 68 | 85-86 | petroleum ether bp 60-80°C | $C_{23}H_{32}O_4$ | 74.16<br>74.14 | 8.66<br>8.57 |

*Table II*

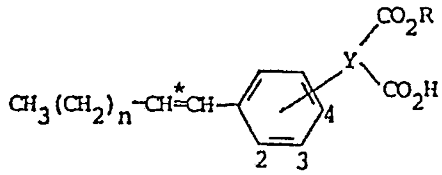

| Ex. No. | n | Y | Position of Y attachment | R | Geometry of *double bond | Yield % | mp, °C | Recrystallization Solvent | Formula | Analysis (calcd/found) C | H |
|---|---|---|---|---|---|---|---|---|---|---|---|
| 29 | 10 | CH=C | 2 | H | Z | 57 | 83-84 | petroleum ether bp 80-100°C | $C_{23}H_{32}O_4$ | 74.16<br>74.06 | 8.67<br>8.34 |
| 30 | 10 | CH=C | 4 | Et | Z | 51 | 86-90 | hexane | $C_{25}H_{36}O_4$ | 74.96<br>74.91 | 9.06<br>9.18 |
| 31 | 8 | $O(CH_2)_2CH$ | 4 | Et | Z | 46 | Oil | — | $C_{24}H_{36}O_5$ | 71.26<br>71.00 | 8.97<br>9.12 |
| 32 | 10 | $O(CH_2)_2CH$ | 2 | Et | Z | 69 | Oil | — | $C_{26}H_{40}O_5$<br>$O:25H_2O$ | 71.44<br>71.45 | 9.34<br>9.31 |
| 33 | 10 | $O(CH_2)_2CH$ | 2 | Et | E | 22 | Oil | — | $C_{26}H_{40}O_5$ | $M^+$ 432.2882 (calcd. $M^+$ 432.2876.) | |

*Example 34:*

*3-(Tridec-1-ynyl) benzyl alcohol*

To a solution of 3-(Tridec-1-ynyl) benzaldehyde (5.68g, 20mmol, from example 5) in THF (70ml) at −78° C was added DIBAL (22ml, 1.1eq, 1 molar solution in hexane). After warming slowly to room temperature the solution was stirred for 2hr and then added to dilute hydrochloric acid (50ml). The product was then extracted into diethyl ether (2×100ml) and after washing with brine and drying ($MgSO_4$) the ether was removed under reduced pressure. Distillation of the resulting oil yielded the desired product (4.55g, 79.5%). B.p. 210° C at 0.1mm Hg, $v_{max}$ 690, 785, 1030, 1460, 1600, 2200, 2850, 2915 and 3300cm$^{-1}$; δ ($CDCl_3$) 0.92 (3H, distorted t, terminal $CH_3$), 1.3 (18H, m, alkylene chain), 1.9 (1H, s, hydroxyl), 2.35 (2H, t, J=7Hz, $CH_2$ adjacent to acetylene), 4.6 (2H, s, benzylics), 7.3 (4H, m, aromatics). $M^+$ 286.2301 ($C_{20}H_{30}O$ requires 286.2297).

Found: C, 83.30, H, 10.53, $C_{20}H_{30}O \cdot ½H_2O$
Requires: C, 83.30, H, 10.57%

*Example 35:*

*3-(Tridec-1-ynyl) benzyl bromide*

Triphenyl phosphine (1.5g, 5.7mmole) was added in small portions to a stirred solution of 3-(Tridec-1-ynyl) benzyl alcohol (1.34g, 4.7mmol, from example 34) and carbon tetrabromide (1.87g, 5.7mmole) in dichloromethane (20ml) at 0° C, and the mixture was stirred for a further 3hr at this temperature. After removing all the solvent under reduced pressure, n-hexane (20ml) was added and the precipitate of triphenylphosphine oxide removed by filtration. Removal of the hexane under reduced pressure gave an oil which was distilled to give the desired product (1.49g, 91%). B.p. 170° C at 0.05mmHg. $v_{max}$ 690, 790, 1210, 1460, 1480, 1600, 2220, 2850, 2920cm$^{-1}$; δ ($CDCl_3$) 0.7 (3H, distorted t, terminal $CH_3$), 1.2 (18H, m, alkylene chain), 2.16 (2H, t, J=7Hz, $CH_2$ adjacent to acetylene), 4.15 (CH, s, benzylics), 7.06 (4H, m, aromatics).

*Example 36:*

*4-(1-Octynyl)benzyl alcohol*

To a solution of 4-(1-octynyl)benzaldehyde (15g, 0.07mole from example 4) in ethanol (200ml) was added sodium borohydride (1.32g, 0.035mole) in ethanol and the mixture was stirred for 5 minutes until no further aldehyde remained. The solution was concentrated, ether was added and the solution was washed with water and dried ($MgSO_4$). Evaporation gave an oil which was chromatographed on silica eluting with dichloromethane to give 12.5g (82%) of the alcohol as an oil, $v_{max}$ (film) 815, 840, 1020, 1040, 1510, 2220, 3300cm$^{-1}$; δ ($CDCl_3$) 0.88 (3H, distorted t, terminal $CH_3$), 1.1-1.8 (8H, m, alkylene chain), 2.2 (1H exchangeable, OH), 2.36 (2H, t, J 7Hz, $CH_2{-}C{\equiv}C$), 4.56 (2H, s, $CH_2O$), 7.3 (4H, ABq, aromatics). $M^+$ 216.1524 ($C_{15}H_{20}O$ requires, 216.1514).

*Example 37:*

*4-(1-Octynyl)benzyl bromide*

Triphenyl phosphine (1.44g, 5.5mmole) was added in small portions to a stirred solution of 4-(1-octynyl)benzyl alcohol (1.0g, 4.6mmole, from example 36) and carbon tetrabromide (1.82g, 5.5mmole) in dichloromethane (20ml) at 0° C, and the mixture was stirred for a further 3 hours at this temperature. Flash chromatography of the concentrated material on silica eluting with dichloromethane: hexane (1:5) and then dichloromethane gave 1.3g (100%) of the bromide as an oil, $v_{max}$ (film) 670, 835, 1200, 1220, 1510, 1610, 2220cm$^{-1}$; δ ($CDCl_3$) 0.8 (3H, distorted t, terminal $CH_3$), 1.7-1.9 (8H, m, alkylene chain), 2.35 (2H, t, J 6Hz, $CH_2C{\equiv}C$), 4.4 (2H, s, $CH_2Br$), 7.3 (4H, s, aromatics). $M^+$ 278.0678 ($C_{15}H_{19}Br$ requires, 278.0670).

Found: C, 64.72, H, 7.05, $C_{15}H_{19}Br$
Requires: C, 64.52, H, 6.85

*Example 38:*

*1-(3-Methoxyphenyl)-oct-1-yne*

3-Methoxyphenylacetylene [5.00g, 0.032M, prepared by the method of E. Negishi *et al*, J. Org. Chem., *45*, 2526-8, (1980)] was dissolved in dry tetrahydrofuran (15ml), cooled to −70° C under nitrogen, and n-butyl lithium, 1.6M in hexane, (24.40ml, 0.038M), added dropwise. After stirring for 0.5h, iodohexane (8.00g, 0.038M), dissolved in dry tetrahydrofuran, (15ml) was added dropwise. The reaction was warmed slowly to room temperature, refluxed overnight, then cooled. The solution was then quenched with water, acidified with dilute hydrochloric acid and extracted with diethyl ether (twice). The organic phases were combined, washed with water and dried ($MgSO_4$). Evaporation *in vacuo* afforded 7.71g of product as an orange oil.

Purification by distillation gave 6.98g (85%) of the product as a colourless oil, bp 150-60° C (Kugelröhr). $v_{max}$ (film) 1465, 1480, 1490 (wk), 1575, 1585, 1600, 1605, 2225 (wk)cm$^{-1}$; δ ($CDCl_3$) 0.88 (3H, distorted t, terminal $CH_3$), 1.37 (8H, m, alkylene chain), 2.36 (2H, t, J 6Hz, acetylenic $CH_2$), 3.74 (3H, s, $OCH_3$), 6.92 (3H, m, aromatics), 7.16 (1H, t, J 7Hz, aromatic).

Found: C, 82.05, H, 9.39, $C_{14}H_{20}O{\cdot}0.25H_2O$
Requires: C, 81.59, H, 9.36%

$M^+$ 216.1504 ($C_{15}H_{20}O$ requires 216.1514).

*Example 39:*

*1-(3-Hydroxyphenyl)-oct-1-yne*

1-(3-Methoxyphenyl)-oct-1-yne (6.50g, 0.03M), was dissolved in 2,4,6-collidine, (100ml), and anhydrous lithium iodide (20.08g, 0.15M), added. The reaction was stirred under nitrogen at reflux temperature for 48h, then cooled and poured into water (500ml), and acidified with dilute hydrochloric acid. The product was extracted into ethyl acetate (twice), and the organic phases combined, washed with water (twice) and dried ($MgSO_4$). Evaporation of the solvent *in vacuo* yielded the crude product as a brown oil, which was purified by chromatography on silica gel, eluting with dichloromethane/n-hexane [1:1] to chloroform. 5.40g (90%) of product was collected as a colourless oil. $v_{max}$ (film) 1580, 1590, 1605, 2230 (weak) 3400cm$^{-1}$; δ ($CDCl_3$) 0.85 (3H, distorted t, terminal $CH_3$), 1.39 (8H, m, alkylene chain), 2.32 (2H, t, J 6Hz, acetylenic $CH_2$), 4.77 (1H, s, OH, exchanged with $D_2O$), 6.78 (2H, m, aromatics), 7.02 (2H, m, aromatics).

Found: C, 84.14, H, 9.01, $C_{14}H_{18}O$
Requires: C, 83.12, H, 8.97%

$M^+$ 202.1357 ($C_{14}H_{18}O$ requires 202.1357).

*Example 40:*

*Diethyl 2-{3-[3-(1-Octynyl)phenoxy]propyl}-propan-1,3-dioate*

1-(3-Hydroxyphenyl)-oct-1-yne (2.20g, 0.01M) and diethyl 2-(3-hydroxypropyl)-propane-1,3-dioate [2.40g, 0.01M, (prepared by the method of A.G. Just *et al*, Tet. Letts., 3645-3648 (1979)] were dissolved in dry tetrahydrofuran (50ml) and triphenylphosphine (4.20g, 0.016M), added. The solution was stirred at 10° C and diethyl azodicarboxylate (2.80g, 0,016M), added dropwise. The reaction was stirred for 0.5h after which time TLC showed some unreacted phenol remaining. Further aliquots of the alcohol (2.40g, 0.01M), triphenylphosphine (4.20g, 0.016M) and diethyl azodicarboxylate (2.80g, 0.016M) were added and the reaction stirred for 0.5h. Evaporation of the solvent *in vacuo* gave a red oil which was purified by chromatography on silica gel eluting with dichloromethane→chloroform, to yield 0.770g (19%) of the title compound as a colourless oil. $v_{max}$ (film) 1580, 1600, 1610, 1740, 1758, 2225 (weak)cm$^{-1}$; δ ($CDCl_3$) 0.87 (3H, distorted t, terminal $CH_3$), 1.26 (6H, t, J 7Hz, 2×ester $CH_3$), 1.33 (8H, m, alkylene chain), 1.93 (4H, m, $OCH_2\underline{CH_2CH_2}$), 2.34 (2H, t, J 6Hz, acetylenic $CH_2$), 3.37 (1H, t, J 7Hz, CH), 3.91 (2H, t, J 6Hz, $OCH_2$), 4.16 (4H, q, J 7Hz, 2×ester $CH_2$), 7.00 (4H, m, aromatics).

Found: C, 70.51, H, 8.46, $C_{24}H_{34}O_5{\cdot}O_{25}H_2O$
Requires: C, 70.82, H, 8.54%

$M^+$ 402.2416 ($C_{24}H_{34}O_5$ requires 402.2406).

*Example 41:*

*1-(2-Hydroxyphenyl)-oct-1-yne*

To a grey suspension of lithium amide [lithium shot, (0.59g, 0.085M) in liquid ammonia (250ml)] at −30° C was added 2-hydroxyphenyl acetylene (50g, 0,042M, prepared by the method of G.A. Russel *et al*, J. Org. Chem., *31*, 248 (1966)) in dry tetrahydrofuran (50ml). The solution was stirred at reflux for 1h, then iodohexane (8.91g, 0.042M), in dry tetrahydrofuran (50ml) was added dropwise. The reaction was refluxed for 6h, then the ammonia allowed to evaporate overnight. The resultant pale brown solution was refluxed for 18h, then cooled and poured into water. Acidified with dilute sulphuric acid and extracted into diethyl ether (twice). The combined organic phases were washed with water (twice), saturated brine and dried ($MgSO_4$). Evaporation *in vacuo* yielded a red oil which was purified by distillation to give 6.80g (80%) of the product as a colourless oil. $\nu_{max}$ (film) 1463, 1485, 1575, 1608 (weak), 2225 (weak), 3500cm$^{-1}$; δ ($CDCl_3$) 0.89 (3H, distorted t, terminal $CH_3$), 1.40 (8H, m, alkylene chain), 2.43 (2H, t, J 7Hz, acetylenic $CH_2$), 5.80 (1H, s, OH), 6.87 (2H, m, aromatics), 7.22 (2H, m, aromatics).

Found: C, 79.64, H, 8.78, $C_{14}H_{18}O \cdot 0.5H_2O$
Requires: C, 79.58, H, 9.06%

$M^+$ 202.1363 ($C_{14}H_{18}O$ requires 202.1358).

*Example 42:*

*(Z) 3-(1-Octenyl)-phenol*

Sodium hydride (3.10g, 0.077M of a 60% dispersion in oil) was washed by decantation with petroleum ether, bp 60-80° C and added to dry, deaerated dimethylsulphoxide (160ml), under nitrogen. The mixture was stirred at 75° C for 0.75h then cooled to 5° C. A solution of n-heptyltriphenylphosphonium bromide (34.0g, 0.077M) in dimethylsulphoxide (40ml) was added and the red solution stirred for 15min. 3-Hydroxybenzaldehyde, sodium salt [9.40g, 0.077M of 3-hydroxy benzaldehyde in dimethylsulphoxide (40ml), treated with one molar equivalent of sodium hydride] was added dropwise at 10° C and the reaction stirred at 10° C for 1h and at room temperature for 3h.

The pale brown mixture was then poured into 10% brine (600ml), acidified with dilute hydrochloric acid and extracted into hexane (twice). The combined organic phases were washed with water (twice), brine and then dried ($MgSO_4$). Evaporation of the solvent and purification by column chromatography using silica gel, eluting with dichloromethane/n-hexane [1:4] yielded 7.1g (45%) of the single Z isomer as a colourless oil. $\nu_{max}$ (film) 1490, 1580, 1590, 1610, 3340cm$^{-1}$; δ ($CDCl_3$) 0.83 (distorted t, terminal $CH_3$), 1.27 (8H, m, alkylene chain), 2.23 (2H, m, allylic $CH_2$), 5.20 (1H, bs, OH, exchanged with $D_2O$), 5.60 (1H, d.t, $J_d$ 11Hz, $J_t$ 7Hz, =$\underline{CH}CH_2$), 6.30 (1H, d, J 11Hz, ArCH=), 6.73 (2H, m, aromatics), 7.16 (1H, m, aromatic), 7.56 (1H, m, aromatic).

Found: C, 80.59, H, 9.57, $C_{14}H_{20}O \cdot 0.25H_2O$
Requires: C, 80.53, H,9.90%

*Table III*

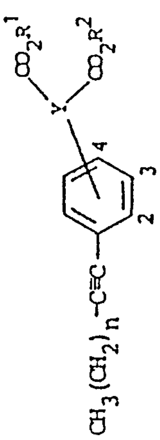

| Example No. | n | Y | Position of Y attachment | $R^1$ | $R^2$ | Yield % | mp, °C | Recrystallization Solvent | Prepared by method of example no. | Formula | Analysis (calcd/found) C | H |
|---|---|---|---|---|---|---|---|---|---|---|---|---|
| 43 | 10 | CH=C | 3 | Et | Et | 76 | oil | — | 1 | $C_{27}H_{38}O_4$ | $M^+$ 426.2780 calcd. 426.2770 | |
| 44 | 5 | CH=C | 4 | Et | Et | 32 | oil | — | 3 | $C_{22}H_{28}O_4$ | $M^+$ 356.1990 calcd. 356.1988 | |
| 45 | 5 | CH=C | 4 | Me | Me | 54 | oil | — | 1 | $C_{20}H_{24}O_4$ | 73.14 73.46 | 7.37 7.51 |

*Table III (continued)*

| Example No. | n | Y | Position of Y attachment | $R^1$ | $R^2$ | Yield % | mp, °C | Recrystallization Solvent | Prepared by method of example no. | Formula | Analysis (calcd/found) C | H |
|---|---|---|---|---|---|---|---|---|---|---|---|---|
| 46 | 10 | $CH_2CH$ | 3 | Et | Et | 45 | oil | — | 12 | $C_{27}H_{40}O_4$ | 75.66<br>75.76 | 9.41<br>9.44 |
| 47 | 5 | $CH_2$ | 4 | Et | Et | 73 | oil | — | 12 | $C_{22}H_{30}O_4$ | 73.71<br>73.92 | 8.43<br>8.32 |
| 48 | 5 | CH=C | 4 | Et | H | 98 | 85-88 | Hexane | 24 | $C_{20}H_{24}O_4$ | 73.14<br>73.11 | 7.37<br>7.44 |
| 49 | 5 | CH=C | 4 | Me | H | 71 | 106-110 | Hexane | 24 | $C_{19}H_{22}O_4$ | 72.59<br>72.33 | 7.05<br>7.20 |
| 50 | 5 | CH=C | 4 | H | H | 63 | 137-138 | Petroleum ether [bp 80-100°C] | 24 | $C_{18}H_{20}O_4$ | 71.98<br>71.65 | 6.71<br>6.72 |
| 51 | 10 | CH=C | 3 | H | H | 76 | 106-107 | Petroleum ether 80-100°C | 24 | $C_{23}H_{30}O_4$ | 74.56<br>74.36 | 8.16<br>8.35 |
| 52 | 10 | $CH_2CH$ | 3 | H | H | 95 | 81-82 | Petroleum ether 80-100°C | 24 | $C_{23}H_{32}O_4$ | 74.16<br>73.93 | 8.66<br>8.92 |
| 53 | 5 | $CH_2CH$ | 4 | H | H | 83 | 115-6 | Petroleum ether 80-100°C | 24 | $C_{18}H_{22}O_4$ | 71.50<br>71.55 | 7.33<br>7.38 |
| 54 | 5 | $O(CH_2)_3CH$ | 2 | Et | Et | 42 | oil | — | 40 | $C_{24}H_{34}O_5 \cdot 0.5H_2O$ | 70.05<br>69.70 | 8.57<br>8.61 |
| 55 | 5 | $O(CH_2)_3CH$ | 2 | Et | H | 52 | oil | — | 24 | $C_{22}H_{30}O_5 \cdot 0.5H_2O$ | 68.93<br>69.01 | 8.15<br>8.17 |
| 56 | 5 | $O(CH_2)_3CH$ | 3 | Et | H | 64 | oil | — | 24 | $C_{22}H_{30}O_5 \cdot 0.25H_2O$ | 69.72<br>69.84 | 8.11<br>7.91 |
| 57 | 5 | $O(CH_2)_3CH$ | 2 | H | H | 93 | oil | — | 24 | $C_{20}H_{26}O_5 \cdot 0.5H_2O$ | 67.58<br>67.30 | 7.66<br>7.92 |
| 58 | 5 | $O(CH_2)_3CH$ | 3 | H | H | 90 | oil | — | 24 | $C_{20}H_{26}O_5$ | 69.34<br>69.64 | 7.57<br>7.47 |
| 73 | 5 | $CHCH_2$ | 4 | Et | H | 63 | oil | — | 24 | $C_{20}H_{26}O_4 \cdot 0.5H_2O$ | 70.77<br>70.38 | 8.02<br>7.80 |

*Table IV*

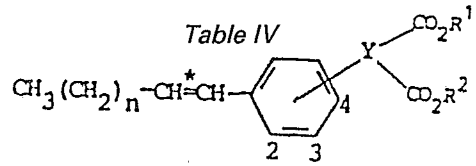

| Example No. | n | Y | Position of Y attachment | $R^1$ | $R^2$ | Geometry of *double bond | Yield % | mp, °C | Recrystallization Solvent | Prepared by method of example no. | Formula | Analysis (calcd/found) C | H |
|---|---|---|---|---|---|---|---|---|---|---|---|---|---|
| 59 | 10 | CH=C | 3 | Et | Et | Z | 54 | oil | — | 10 | $C_{27}H_{40}O_4$ | 75.66<br>75.59 | 9.40<br>9.50 |
| 60 | 5 | CH=C | 4 | Me | Me | Z | 62 | oil | — | 10 | $C_{20}H_{26}O_4$ | 73.15<br>73.46 | 7.30<br>7.30 |
| 61 | 5 | CH=C | 4 | Et | Et | Z | 82 | oil | — | 10 | $C_{22}H_{30}O_4$ | 73.71<br>73.55 | 8.43<br>8.42 |
| 62 | 5 | CH=C | 4 | Me | H | Z | 63 | 57-60 | Hexane | 24 | $C_{19}H_{24}O_4$ | 72.12<br>71.84 | 7.60<br>7.50 |
| 63 | 10 | CH=C | 3 | H | H | Z | 93 | 78-79 | Petroleum ether bp 80-100°C | 24 | $C_{23}H_{32}O_4 \cdot 0.25H_2O$ | 73.29<br>73.12 | 8.60<br>8.50 |
| 64 | 5 | CH=C | 4 | H | H | Z | 100 | 72-75 | — | 24 | $C_{18}H_{22}O_4 \cdot 0.25H_2O$ | 70.91<br>70.85 | 6.70<br>7.20 |
| 65 | 10 | $CH_2CH$ | 3 | Et | Et | Z | 72 | oil | — | 10 | $C_{27}H_{42}O_4$ | $M^+$ 430.3089<br>calcd. 430.3083 | |
| 66 | 10 | $CH_2CH$ | 3 | H | H | Z | 95 | 57-58 | Hexane | 24 | $C_{23}H_{34}O_4$ | 73.76<br>73.45 | 9.10<br>9.20 |
| 67 | 5 | $O(CH_2)_3CH$ | 3 | Et | Et | Z | 71 | oil | — | 40 | $C_{24}H_{36}O_5$ | 71.26<br>71.28 | 8.90<br>9.00 |
| 68 | 5 | $O(CH_2)_3CH$ | 3 | Et | H | Z | 99 | oil | — | 24 | $C_{22}H_{32}O_5$ | 70.18<br>69.98 | 8.50<br>8.10 |
| 69 | 5 | $O(CH_2)_3CH$ | 3 | H | H | Z | 90 | 71 | Hexane | 24 | $C_{20}H_{28}O_5 \cdot 0.25H_2O$ | 68.05<br>68.25 | 8.00<br>8.00 |
| 70 | 5 | $CH_2CH$ | 4 | Et | Et | Z | 85 | oil | — | 10 | $C_{22}H_{32}O_4$ | $M^+$ 360.2306<br>calcd. 360.2310 | |
| 71 | 5 | $CH_2CH$ | 4 | Et | H | Z | 34 | oil | — | 24 | $C_{20}H_{26}O_4 \cdot 0.5H_2O$ | 70.77<br>70.38 | 8.00<br>7.00 |
| 72 | 5 | $CH_2CH$ | 4 | H | H | Z | 74 | 82-85 | Hexane | 24 | $C_{18}H_{24}O_4$ | 71.02<br>71.11 | 7.00<br>7.00 |

## PHARMACOLOGICAL DATA

*1) RBL-1 5-lipoxygenase Screen*

5-Lipoxygenase enzyme was prepared as a 10,000g supernatant from RBL-1 cells by the method of Jakschik (Jakschik, B.A., F.F. Sun, L.M. Lee, and M.M. Steinhoff, 1980, Biochem. Biophys. Res. Comm., *95*, 103). The 10,000g supernatant was diluted with homogenization buffer to the equivalent of $1.5\text{-}2.5\times10^7$ cells. $ml.^{-1}$ and made 2mM with respect to $CaCl_2$. Aliquots of 0.5ml were then dispensed into tubes, and incubated at 29° C with 5µl ethanol or compound in ethanol at the desired concentration for 2min. Then [$1\text{-}^{14}C$] arachidonic acid was added in buffer to give a final concentration of 6.3µM and 0.2µCi per incubation, and the reaction continued at 29° C for 2min. The reaction was terminated by adding 1 ml of acetone and cooling on ice, 0.5ml of ice-cold saline and 100µl of 2M formic acid were added, and the mixture was extracted with 2×2ml of chloroform. The extract was stored under $N_2$ at −20° C until analysis by chromatography. Activity was measured as the percentage of total radioactivity found in 5-HETE and 5,12-diHETE, and inhibition calculated as the decrease in formation of the sum of these two species in compound-treated incubates relative to control incubations.

*2) Cyclo-oxygenase Screen*

The inhibition of cyclo-oxygenase was measured in a buffered incubation (0.2M Tris-HCl, pH 8.0, containing 1mM ethylene diaminetetraacetic acid) comprising 0.96mg lyophilised bovine seminal vesicle microsomes, 5-15µM arachidonic acid containing 0.2µCi [$1\text{-}^{14}C$] arachidonic acid, 2mM reduced glutathione, 0.5mM hydroquinone, 1µM haemoglobin and compound (0-0.05mM in 5µl dimethylsulphoxide or absolute ethanol) in a total volume of 2.0ml. Compounds were preincubated with the incubation constituents for 5min at 37° C before the reaction was started by the addition of the arachidonic acid. The reaction was continued at 37° C for 2min, then stopped by placing the incubations on ice and the addition of 1.2ml 0.2M citric acid. Unmetabolised substrate and prostaglandin products were extracted in ethyl acetate (2×4ml), the combined extracts washed with 0.8ml water, and separated by thin-layer chromatography (Kieselgel $GF_{254}$ plates in ethyl acetate:acetone:glacial acetic acid, 90:10:1, v/v). Recovery was 65-80%. The regions on the thin-layer chromatography plate that chromatographed with authentic arachidonic acid or prostaglandins $E_2$ and $F_2\alpha$ ($R_f$'s 0.70, 0.28 and 0.16 respectively) were scraped and the radioactivity in each determined by liquid scintillation counting with a correction for quenching being made by the external standard-channels ratio method. Inhibition of cyclo-oxygenase activity was calculated from the decrease in prostaglandin formation. Each compound concentration was tested in triplicate and the 50% inhibitory concentration, if determined, was calculated by linear regression from the inhibitory data at, at least three different compound concentrations.

## BIOLOGICAL RESULTS

| Example No. | 5-Lipoxygenase Inhibition at 20µM | Cyclo-Oxygenase Inhibition at 50µM |
|---|---|---|
| 24 | 60* | 70 |
| 25 | 55* | 59 |
| 27 | | 59 |
| 28 | 63 | 76 |
| 29 | 67 | |
| 49 | 15 | |
| 50 | 16 | |
| 51 | 25 | |
| 52 | 51 | |
| 55 | 63 | |
| 56 | 52* | |
| 57 | 54 | |
| 58 | 45* | 38 |
| 63 | 82 | |
| 64 | 49 | |
| 66 | 84 | |
| 68 | 62.5 | 40 |
| 69 | 44 | |
| 71 | 30 | |

* Indicates inhibition at 50µM

## Claims

1. A compound of formula (I):

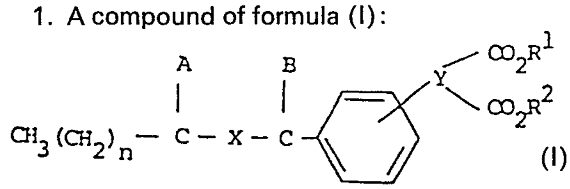

(I)

or a salt thereof,
in which Y is a group

$-O(CH_2)_mCH<$ , $-(CH_2)_mCH<$ , or $-CH=C<$

where m is an integer of from 1 to 5,
n is an integer of from 4 to 12,
each of $R^1$ and $R^2$, which may be the same or different, represents hydrogen or $C_{1-6}$ alkyl,
X represents a double or triple bond, and each of A and B represents hydrogen when X is a double bond, or both A and B are absent when X is a triple bond.

2. A compound according to claim 1, in which the substituents on the aromatic ring are in the 1,2- or 1,3- position.

3. A compound according to claim 1 or claim 2, in which n is an integer from 8 to 12.

4. A compound according to any one of claims 1 to 3, in which m is 1, 2 or 3.

5. A compound according to any one of claims 1 to 4 in which, when X is a double bond, the hydrocarbon chain containing X has the (Z) absolute configuration.

6. A compound according to claim 1, selected from

2-[2-(1-tridecynyl)phenyl]methylenepropan-1,3-dioic acid mono ethyl ester;

2-[2-(1-tridecynyl)phenyl]methylenepropan-1,3-dioic acid;
2-[2-(1-tridecynyl)phenyl]methylpropan-1,3-dioc acid;
2-[2-[(Z)-1-tridecenyl]phenyl]methylene-propan-1,3-dioic acid;
2-[3-(1-tridecynyl)phenyl]methylenepropan-1,3-dioic acid;
2-[3-(1-tridecynyl)phenyl]methylpropan-1,3-dioic acid;
2-[3-[2-(1-octynyl)phenoxy]propyl]propan-1,3-dioic acid mono ethyl ester;
2-[3-[3-(1-octynyl)phenoxy]propyl]propan-1,3-dioic acid mono ethylester;
2-[3-[2-(1-octynyl)phenoxy]propyl]propan-1,3-dioic acid;
2-[3-[3-(1-octynyl)phenoxy]propyl]propan-1,3-dioic acid;
2-[3-[(Z)-1-tridecenyl]phenyl]methylene-propan-1,3-dioic acid;
2-[3-[(Z)-1-tridecenyl]phenyl]methylpropan-1,3-dioic acid;
2-[3-[3-([Z]-1-octenyl)phenoxy]-propyl]-propan-1,3-dioic acid mono ethyl ester;
2-[3-[3-([Z]-1-octenyl)phenoxy]propyl]-propan-1,3-dioic acid.

7. A process for preparing a compound according to claim 1, which comprises

(a) treating a compound of formula (II),

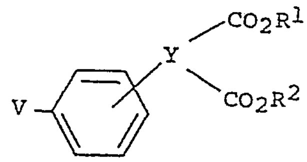

(II)

in which Y is as defined with reference to formula (I) in claim 1, each of $R^1$ and $R^2$ are $C_{1-6}$ alkyl, and

V represents 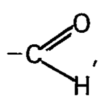, $-\overset{\ominus}{C}H-\overset{\oplus}{P}Ph_3$ or halogen,

with a compound of formula (III),

$$CH_3(CH_2)_n-W \quad (III)$$

in which W is 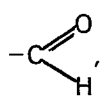, $-\overset{\ominus}{C}H-\overset{\oplus}{P}Ph_3$, $-CH=CH_2$ or $-C{\equiv}CH$,

and, optionally thereafter reducing the carbon-carbon triple bond, when present in the resultant product, to a carbon-carbon double bond, and/or optionally converting the resultant product to the corresponding mono- or dibasic acid, with the provisos that

i) when V is 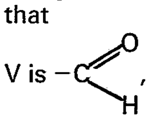, W is $-\overset{\ominus}{C}H-\overset{\oplus}{P}Ph_3$,

ii) when V is $-\overset{\ominus}{C}H-\overset{\oplus}{P}Ph_3$, W is 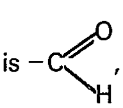, and

iii) when V is halogen, W is $-CH=CH_2$ or $-C{\equiv}CH$, or

(b) treating a compound of formula (IV)

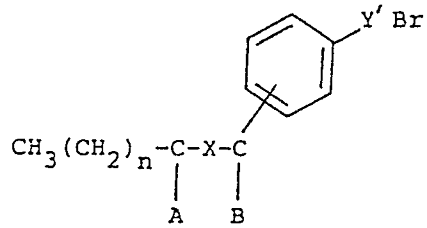

(IV)

in which n, A, B and X are as defined in formula (I) in claim 1, and Y' is $-O(CH_2)_m-$ or $-(CH_2)_m-$, with a malonic diester of formula (V)

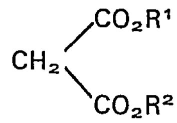

(V)

in which $R^1$ and $R^2$ are each $C_{1-6}$ alkyl, or

(c) treating a compound of formula (VIII)

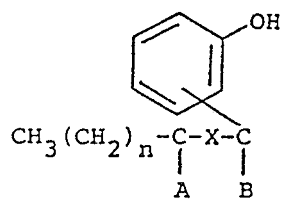

(VIII)

in which n, A, B and X are as defined in formula (I) in claim 1,
with a compound of formula (IX)

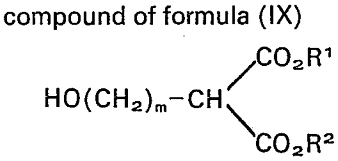

(IX)

in which m, $R_1$ and $R_2$ are as defined in formula (I).

8. A pharmaceutical composition comprising a compound according to any one of claims 1 to 6, and a pharmaceutically acceptable carrier.

9. A compound according to any one of claims 1 to 6, or a composition according to claim 8, for use in the therapeutic or prophylactic medical treatment of human or non-human animals.

10. A compound according to any one of claims 1 to 6, or a composition according to claim 8, for use in the treatment of allergic diseases in human or non-human animals.

## Patentansprüche

1. Eine Verbindung der Formel (I):

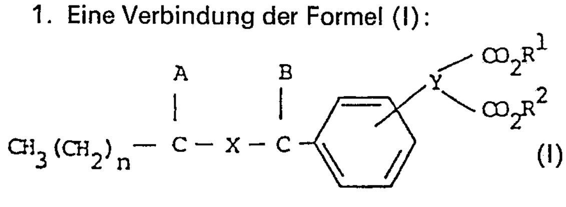

(I)

oder ein Salz derselben,
in welcher Y eine Gruppe

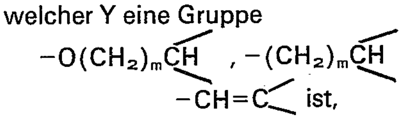 oder ist,

wobei m eine ganze Zahl von 1 bis 5 bedeutet, n eine ganze Zahl von 4 bis 12 ist, jede der beiden Gruppen $R^1$ und $R^2$, welche gleich oder verschieden sein können, Wasserstoff oder $C_{1-6}$-Alkyl darstellt, X eine Doppel- oder Dreifachbindung darstellt und jede der beiden Gruppen A und B Wasserstoff bedeutet, wenn X eine Doppelbindung ist, oder beide Gruppen A und B nicht vorhanden sind, wenn X eine Dreifachbindung darstellt.

2. Eine Verbindung gemäss Anspruch 1, in welcher die Substituenten am aromatischen Ring sich in der 1,2- oder 1,3- Stellung befinden.

3. Eine Verbindung gemäss Anspruch 1 oder Anspruch 2, in welcher n eine ganze Zahl von 8 bis 12 ist.

4. Eine Verbindung gemäss einem der Ansprüche 1 bis 3, in welcher m den Wert 1, 2 oder 3 hat.

5. Eine Verbindung gemäss einem der Ansprüche 1 bis 4, in welcher, wenn X eine Doppelbindung ist, die Kohlenwasserstoffkette, welche X enthält, die absolute Konfiguration (Z) besitzt.

6. Eine Verbindung gemäss Anspruch 1, ausgewählt aus

2-[2-(1-Tridecynyl)phenyl]methylenpropan-1,3-dicarbonsäure-monoäthylester;

2-[2-(1-Tridecynyl)phenyl]methylenpropan-1,3-dicarbonsäure;

2-[2-(1-Tridecynyl)phenyl]methylpropan-1,3-dicarbonsäure;

2-[2-[(Z)-1-tridecenyl]phenyl]methylenpropan-1,3-dicarbonsäure;

2-[3-(1-Tridecynyl)phenyl]methylenpropan-1,3-dicarbonsäure;

2-[3-(1-Tridecynyl)phenyl]methylpropan-1,3-dicarbonsäure;

2-[3-[2-(1-Octynyl)phenoxy]propyl]propan-1,3-dicarbonsäure-monoäthylester;

2-[3-[3-(1-Octynyl)phenoxy]propyl]propan-1,3-dicarbonsäure-monoäthylester;

2-[3-[2-(1-Octynyl)phenoxy]propyl]propan-1,3-dicarbonsäure;

2-[3-[3-(1-Octynyl)phenoxy]propyl]propan-1,3-dicarbonsäure;

2-[3-[(Z)-1-Tridecenyl]phenyl]methylenpropan-1,3-dicarbonsäure;

2-[3-[(Z)-1-Tridecenyl]phenyl]methylpropan-1,3-dicarbonsäure;

2-[3-[3-([Z]-1-Octenyl)phenoxy]-propyl]-propan-1,3-dicarbonsäuremonoäthylester;

2-[3-[3-([Z]-1-Octenyl)phenoxy]propyl]-propan-1,3-dicarbonsäure.

7. Ein Verfahren zur Herstellung einer Verbindung gemäss Anspruch 1, welches

(a) das Behandeln einer Verbindung der Formel (II),

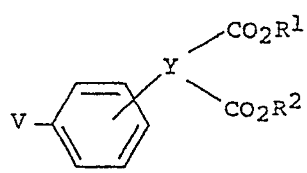

(II)

in welcher Y wie in bezug auf Formel (I) in Anspruch 1 definiert ist, jede der beiden Gruppen $R^1$ und $R^2$ $C_{1-6}$-Alkyl bedeutet, und V eine Gruppe

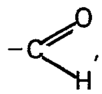 , $-\overset{\ominus}{C}H-\overset{\oplus}{P}Ph_3$ oder ein

Halogen darstellt, mit einer Verbindung der Formel (III)

$$CH_3(CH_2)_n-W \qquad (III)$$

in welcher W eine Gruppe

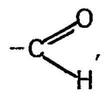 , $-\overset{\ominus}{C}H-\overset{\oplus}{P}Ph_3$,

$-CH=CH_2$ oder $-C\equiv CH$ ist, und gegebenenfalls anschliessend die Reduktion der Kohlenstoff-Kohlenstoff-Dreifachbindung, falls im Endprodukt vorhanden, zu einer Kohlenstoff-Kohlenstoff-Doppelbindung, und/oder gegebenenfalls die Umwandlung des Endprodukts in die entsprechende mono- oder dibasische Säure, mit der Massgabe dass

i) wenn V die Gruppe 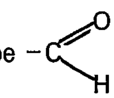 ist, W die Gruppe $-\overset{\ominus}{C}H-\overset{\oplus}{P}Ph_3$ bedeutet,

ii) wenn V die Gruppe $-\overset{\ominus}{C}H-\overset{\oplus}{P}Ph_3$ ist W die Gruppe 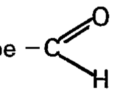 bedeutet, und

iii) wenn V ein Halogen ist, W eine Gruppe $-CH=CH_2$ oder $-C\equiv CH$ ist, oder

(b) das Behandeln einer Verbindung der Formel (IV)

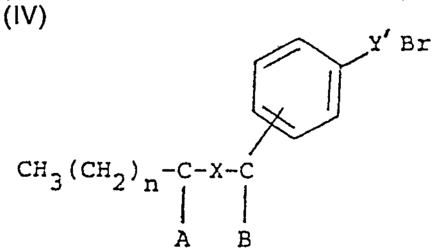

(IV)

in welcher n, A, B und X wie in bezug auf Formel (I) in Anspruch 1 definiert sind, und Y' $-O(CH_2)_m-$ oder $-(CH_2)_m-$ bedeutet, mit einem Malonsäurediester der Formel (V)

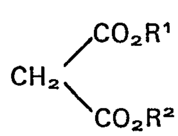

(V)

in welcher $R^1$ und $R^2$ jeweils $C_{1-6}$-Alkyl sind, oder

(c) das Behandeln einer Verbindung der Formel (VIII)

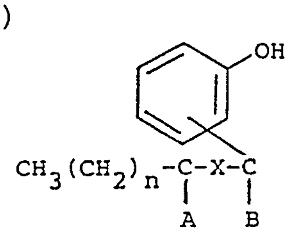

(VIII)

in welcher n, A, B und X wie in bezug auf Formel (I) in Anspruch 1 definiert sind, mit einer Verbindung der Formel (IX)

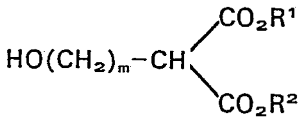

(IX)

in welcher m, $R_1$ und $R_2$ wie in bezug auf Formel (I) definiert sind, umfasst.

8. Eine pharmazeutische Zusammensetzung umfassend eine Verbindung gemäss einem der Ansprüche 1 bis 6, und einen pharmazeutisch verträglichen Träger.

9. Eine Verbindung gemäss einem der Ansprüche 1 bis 6, oder eine Zusammensetzung gemäss Anspruch 8, zur Verwendung bei der therapeutischen oder vorsorglichen medizinischen Behandlung von Mensch und Tier.

10. Eine Verbindung gemäss einem der Ansprüche 1 bis 6 oder eine Zusammensetzung gemäss Anspruch 8, zur Verwendung bei der Behandlung von Allergiekrankheiten bei Mensch und Tier.

## Revendications

1. Composé de formule (I):

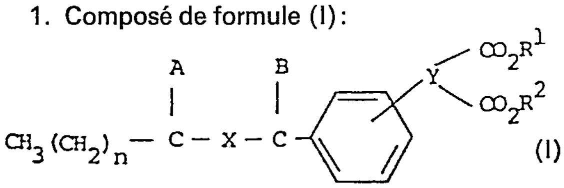

(I)

ou un sel de celui-ci
dans laquelle Y est un groupe

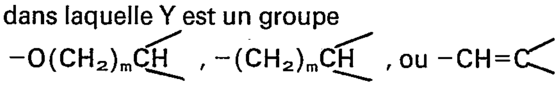

où m est un entier de 1 à 5,
n est un entier de 4 à 12,
chacun des $R^1$ et $R^2$ pouvant être identique ou différent, représente un atome d'hydrogène ou un groupe alcoyle en $C_{1-6}$,
X représente une liaison double ou triple et chacun des A et B représente un atome d'hydrogène lorsque X est une double liaison, ou A et B sont tous deux absents lorsque X est une triple liaison.

2. Composé suivant la revendication 1, dans lequel les substituants portés sur le noyau aromatique sont en position 1,2 ou 1,3.

3. Composé suivant la revendication 1 ou la revendication 2, dans lequel n est un entier de 8 à 12.

4. Composé suivant l'une quelconque des revendications 1 à 3, dans lequel m est 1, 2 ou 3.

5. Composé suivant l'une quelconque des revendications 1 à 4, dans lequel, dans le cas où X est une double liaison, la chaîne hydrocarbonée contenant X a la configuration absolue (Z).

6. Composé suivant la revendication 1, choisi parmi:
l'ester monoéthylique de l'acide 2-[2-(1-tridécynyl)phényl]méthylènepropan-1,3-dioïque;
l'acide 2-[2-(1-tridécynyl)phényl]méthylènepropan-1,3-dioïque;
l'acide 2-[2-(1-tridécynyl)phényl]méthylpropan-1,3-dioïque;
l'acide 2-[2-[(Z)-1-tridécényl]phényl]méthylènepropan-1,3-dioïque;
l'acide 2-[3-(1-tridécynyl)phényl]méthylènepropan-1,3-dioïque;
l'acide 2-[3-(1-tridécynyl)phényl]méthylpropan-1,3-dioïque;
l'ester monoéthylique de l'acide 2-[3-[2-(1-octynyl)phénoxy]propyl]propan-1,3-dioïque;
l'ester monoéthylique de l'acide 2-[3-[3-(1-octynyl)phénoxy]propyl]propan-1,3-dioïque;
l'acide 2-[3-[2-(1-octynyl)phénoxy]propyl]-propan-1,3-dioïque;
l'acide 2-[3-[3-(1-octynyl)phénoxy]propyl]-propan-1,3-dioïque;
l'acide 2-[3-[(Z)-1-tridécényl]phényl]méthylènepropan-1,3-dioïque;
l'acide 2-[3-[(Z)-1-tridécényl]phényl]méthylpropan-1,3-dioïque;
l'ester monoéthylique de l'acide 2-[3-[3-([Z]-1-octényl)phénoxy]-propyl]propan-1,3-dioïque; et
l'acide 2-[3-[3-([Z]-1-octényl)phénoxy]-propyl]propan-1,3-dioïque.

7. Procédé de préparation d'un composé suivant la revendication 1 qui comprend:
(a) le traitement d'un composé de formule (II)

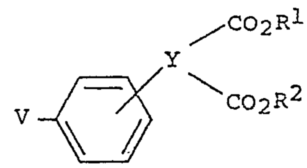

(II)

dans laquelle Y est tel que défini à propos de la formule (I) dans la revendication 1, chacun des $R^1$ et $R^2$ est un groupe alcoyle en $C_{1-6}$ et V représente

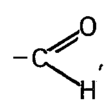, $-\overset{\ominus}{C}H-\overset{\oplus}{P}Ph_3$

ou un atome d'halogène,
avec un composé de formule (III)

$$CH_3(CH_2)_n-W \quad (III)$$

dans laquelle W est 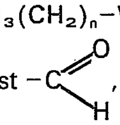, $-\overset{\ominus}{C}H-\overset{\oplus}{P}Ph_3$, $-CH=CH_2$ ou $-C\equiv CH$,
et éventuellement ensuite, la réduction de la triple liaison carbone-carbone lorsqu'elle est présente dans le produit résultant en une double liaison et/ou la conversion éventuelle du produit résultant en l'acide mono- ou difonctionnel correspondant, avec les conditions suivantes:

(i) lorsque V est 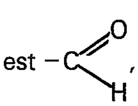, W est $-\overset{\ominus}{C}H-\overset{\oplus}{P}Ph_3$,

(ii) lorsque V est $-\overset{\ominus}{C}H-\overset{\oplus}{P}Ph_3$, W est 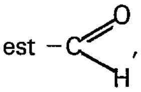, et

(iii) lorsque V est un atome d'hydrogène, W est $-CH=CH_2$ ou $-C\equiv CH$, ou

(b) le traitement d'un composé de formule (IV)

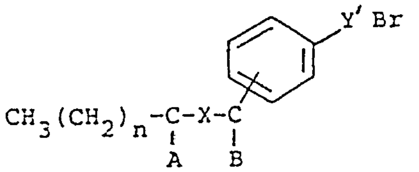

(IV)

dans laquelle n, A, B et X sont tels que définis à propos de la formule (I) dans la revendication 1 et Y' est $-O(CH_2)_m-$ ou $-(CH_2)_m-$, avec un diester malonique de formule (V)

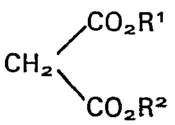

(V)

dans laquelle $R^1$ et $R^2$ sont chacun un groupe alcoyle en $C_{1-6}$, ou

(c) le traitement d'un composé de formule (VIII)

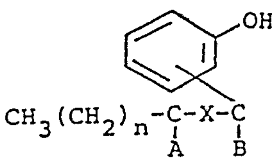

(VIII)

dans laquelle n, A, B et X sont tels que définis dans la formule (I) dans la revendication 1, avec un composé de formule (IX)

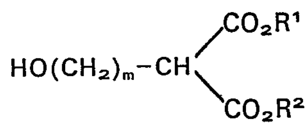

(IX)

dans laquelle m, $R_1$ et $R_2$ sont tels que définis dans la formule (I).

8. Composition pharmaceutique comprenant un composé suivant l'une quelconque des revendications 1 à 6, et un support acceptable du point de vue pharmaceutique.

9. Composé suivant l'une quelconque des revendications 1 à 6, ou composition suivant la revendication 8, utile dans le traitement médical thérapeutique ou prophylactique de l'homme ou d'animaux non humains.

10. Composé suivant l'une quelconque des revendications 1 à 6, ou composition suivant la revendication 8, utile dans le traitement de maladies allergiques chez l'homme ou des animaux non humains.